# EUROPÄISCHE PATENTSCHRIFT

(11) **EP 2 263 739 B1**
(45) Veröffentlichungstag und Bekanntmachung des Hinweises auf die Patenterteilung: **18.01.2012**
(21) Anmeldenummer: 10006203.3
(22) Anmeldetag: 15.06.2010
(51) Int. Cl.: A61M 37/00, A61K 9/00

(54) **Medizinisches System**
Medical system
Système médical

(30) Priorität: 15.06.2009 DE 102009025297
(43) Veröffentlichungstag der Anmeldung: 22.12.2010
(73) Patentinhaber: Heraeus Medical GmbH, 61273 Wehrheim (DE)
(72) Erfinder: Bösebeck, Hans, Dr., 63775 Alzenau-Wasserlos (DE); Vogt, Sebastian, Dr., 99092 Erfurt (DE); Büchner, Hubert, Dr., 64354 Reinheim (DE)
(74) Vertreter: Kühn, Hans-Christian

(56) Entgegenhaltungen:
- WO-A2-2006/091744
- US-A1- 2005 278 023
- US-A1- 2007 162 132
- US-B1- 6 183 768
- US-B1- 6 299 590

## Beschreibung

Die Erfindung betrifft ein medizinisches System mit den Merkmalen des Oberbegriffs des Patentanspruchs 1.

Die Deutsche Offenlegungsschrift DE 2 320 373 A sowie die US 6,183,768 B1, die US 6,299,590 B1 und die WO 2006/091744 A2 beschreiben Wirkstoffketten, die mit einem Antibiotikum beladen sind und in Wundhöhlen eingelegt werden können. Solcherart Wirkstoffketten bestehen häufig aus einem PMMA-Kunststoff (Polymethylmethacrylat), welcher mit inkorporierten Antibiotika versehen ist. Diese Wirkstoffketten werden etwa bei Hüftoperationen neben das künstliche Hüftgelenk gelegt, um etwaigen Entzündungen vorzubeugen. Allerdings müssen je nach Indikation die Wirkstoffketten nach einigen Tagen bis Wochen wieder entfernt werden. Diese Entfernung ist jedoch mitunter sehr schwierig, da die Wirkstoffketten durch neu gebildetes Bindegewebe eingeschlossen sind.

Im Stand der Technik ist es bekannt die Wirkstoffketten zum Entfernen an einem herausragenden Ende mit einer Pinzette, einer Komzange oder einem Nagelhalter zu fassen und händisch herauszuziehen. Aus der US 2005/278023 A1 und US 20071162132 A1 sind zahnradartige Systeme bekannt, um Wirkstoffketten zu entfernen. Im Stand der Technik kommt es vielfach zum Abreißen und/oder Zersplittern von einzelnen Elementen der Wirkstoffketten. In solch einem Falle ist der Chirurg gezwungen, die Wunde weiter zu öffnen, um dann noch an Reste der Wirkstoffkette zu gelangen. Dadurch können neue infekte auftreten und/oder der Hellprozess des Patienten verlängert werden.

Es ist Aufgabe der vorliegenden Erfindung, ein medizinisches System zu schaffen, welches die vorgenannten Nachteile vermeidet, insbesondere ein Zerreißen und/oder Zersplittern der Wirkstoffkette verhindert.

Zur Lösung dieser Aufgabe wird ein medizinisches System mit den Merkmalen des Anspruchs 1 vorgeschlagen. In den abhängigen Ansprüchen sind bevorzugte Weiterbildungen aufgeführt.

Im Rahmen der Erfindung wird ein medizinisches System mit einer Zugvorrichtung und einer Wirkstoffkette beschrieben, wobei die Wirkstoffkette mindestens zwei Speichermittel aufweist, die kettenartig angeordnet sind, wenigstens eines der Speichermittel mit einem medizinischen Wirkstoff beladen ist, die Zugvorrichtung ein Griffelement und ein Koppelelement aufweist, und das Koppelelement wenigstens eine Aussparung aufweist, in einer Freilage die Wirkstoffkette getrennt von der Zugvorrichtung angeordnet ist, in einer Einschublage zumindest eines der Speichermittel formschlüssig in der Aussparung angeordnet ist, und die Wirkstoffkette reversible aus der Freilage in die Einschublage überführbar ist.

Der Hauptgedanke der Erfindung besteht in einer Zugvorrichtung für die Wirkstoffkette, die zumindest eines der Speichermittel zumindest bereichsweise formschlüssig in einer Aussparung umfasst. Im Gegensatz zu den bekannten Mitteln, die für das Ziehen der Wirkstoffkette benutzt werden, weist die erfindungsgemäße Zugvorrichtung eine Aussparung auf, deren Größe und Formgebung derart ist, dass sie zumindest Teile des Speichermittels der Wirkstoffkette formschlüssig umgreift. Somit wird eine punktuelle Belastung der Wirkstoffkette vermieden. Vielmehr sorgt eine großflächige Koppelung zwischen der Zugvorrichtung und der Wirkstoffkette dafür, dass an keiner Stelle punktuell hohe Kräfte auf die Wlrkstoffkette einwirken. Im Rahmen des Ziehens einer Wirkstoffkette aus einer Wunde können Kräfte bis zu 300 N auftreten. Durch die formschlüssige Umfassung zumindest eines Speichermittels in wenigstens einer Aussparung wird sichergestellt, dass diese Kraft nicht punktuell auf einen kleinen Bereich des Speichermittels wirkt. Vielmehr wird eine größere, insbesondere halbkugelartig geformte Fläche des Speichermittels von einem ebenso geformten Bereich der Aussparung umfasst. Der so sich ergebende Formschluss zwischen dem Speichermittel und der Aussparung sorgt dafür, dass keine Zerstörungen der Wirkstoffkette mehr auftreten können, wenn diese aus der Wunde gezogen wird.

Um eine sichere Lagerung der Wirkstoffkette in der Einschublage in der Zugvorrichtung zu gewährleisten, hat es sich als vorteilhaft herausgestellt, die wenigstens eine Aussparung zylinderartig oder kugelartig auszuformen. Diese Ausformung der Aussparung ist besonders gut geeignet, ein kugelartig ausgeformtes Speichermittel aufzunehmen. Jenes kugelartige Speichermittel kann dann in die Aussparung hineinrutschen, ohne dass die Gefahr eines Verkantens besteht. Weiterhin hat es sich als vorteilhaft herausgestellt, wenn ein Durchmesser der wenigstens einen Aussparung größer ist als der Durchmesser eines Speichermittels. Dadurch ist ein leichtes und einfaches Einführen der Speichermittel in die Aussparung sichergestellt. Zusätzlich können sich Flüssigkeiten, wie etwa Blut, auf dem Speichermittel abgesetzt haben, so dass eine Aussparung, deren Durchmesser exakt jenem des Speichermittels entspricht, nicht ausreicht, um das Speichermittel aufzunehmen. Vorteilhafterweise ist der Durchmesser der Aussparung 2 % bis 10 % größer als der Durchmesser des Speichermittels.

Um bei längeren Wirkstoffketten ein einfaches Nachfassen zu ermöglichen, hat es sich als vorteilhaft herausgestellt, wenn das Koppelelement ein Durchgangselement aufweist, welches eine Durchführung in dem Koppelelement und/oder der Zugvorrichtung bildet. Durch dieses Durchgangselement können Teile der Wirkstoffkette geführt werden. Hat der Operateur folglich die Wirkstoffkette in der Einschublage ein Stück aus der Wunde herausgezogen, kann er die Speichermittel aus den Aussparungen aushaken. Die Wirkstoffkette befindet sich dann in der Freilage. Nunmehr ist es dem Operateur möglich, die Wirkstoffkette durch das Durchgangselement hindurch zu fädeln und Speichermittel, die näher an der Wunde liegen, wieder in die Aussparung einzuführen - folglich die Wirkstoffkette wieder in die Einschublage zu überführen. Sollte solch ein Nachfassen noch einmal nötig sein, würde der Operateur nicht mehr gezwungen sein, die Wirkstoffkette in die Freilage zu überführen. Vielmehr würde es reichen, die Speichermittel aus den Aussparungen herauszuziehen und das Koppelelement derart über die Wirkstoffkette zu schieben, dass dieses sich weiter in Richtung der Wunde bewegt. Dann könnte er die Speichermittel wieder in die Aussparung einführen. Dadurch, dass die Wirkstoffkette durch das Durchgangselement läuft, kann die Zugvorrichtung und/oder das Koppelelement über die Wirkstoffkette longitudinal bewegt werden. Dabei hat es sich als besonders vorteilhaft herausgestellt, wenn das Durchgangselement zwischen der wenigstens einen Aussparung und dem Griffelement angeordnet ist und/oder durch das Griffelement hindurchläuft. Dies ermöglicht ein leichtes Bewegen der Zugvorrichtung über die Wirkstoffkette.

Da das Durchgangselement nicht dazu dient, Kräfte auf die Wirkstoffkette zu überführen, hat es sich als vorteilhaft erwiesen, wenn das Durchgangselement einen Durchmesser aufweist, der größer ist als der Durchmesser der wenigstens einen Aussparung. Der Durchmesser des Durchgangselementes liegt vorteilhafterweise 10 % bis 15 % über dem Durchmesser des Speichermittels. So ist ein glattes Durchführen der Wirkstoffkette durch das Durchgangselement sichergestellt. Insbesondere hat es sich bei kugelartigen Speichermitteln als vorteilhaft herausgestellt, wenn das Durchgangselement zylinderartig ausgeformt ist.

In einer bevorzugten Ausführungsvariante weist die Zugvorrichtung mehr als zwei Aussparungen auf. Bevorzugt sind zwei oder drei Aussparungen, die in einer Reihe auf dem Koppelelement angeordnet sind. Durch die Wahl einer Mehrzahl von Aussparungen reduziert sich die auf ein einzelnes Speichermittel in der Einschublage wirkende Kraft anteilig. Folglich wird die Wahrscheinlichkeit für ein Zersplittern einer der Wirkstoffkugeln und/oder ein Zerstören der Wirkstoffkette weiter reduziert.

Um eine Selbstarretierung des Speichermittels in der Aussparung zu erreichen, zeichnet sich die Ausführungsvariante dadurch aus, dass eine Mittelachse der wenigstens einen Aussparung gegenüber einer von dem Griffelement und dem Koppelelement aufgespannten Ebene geneigt ist. Dabei ist es besonders vorteilhaft, wenn die Mittelachse gegenüber der aufgespannten Ebene in Richtung des Griffelementes geneigt ist. Soll eine sich in einem Körper befindliche Wirkstoffkette herausgezogen werden, wird das Speichermittel in die Einschublage überführt. Im Anschluss zieht der Operateur an dem Griffelement der Zugvorrichtung. Da die Wrkstoffkette zum Teil eingewachsen ist in dem Knochen und/oder dem Gewebe des Patienten muss er eine Kraft aufbringen, die dazu führt, dass das Speichermittel durch die geneigte Aussparung in das Koppelelement hineingezogen wird. Ein Boden der Aussparung kommt dann in Kontakt mit dem Speichermittel, welches durch die beim Ziehen aufgebrachte Kraft gegen diesen Boden gepresst wird. Als Resultat arretiert sich das Speichermittel der Wirkstoffkette selbst in der Aussparung. Dies hat den Vorteil, dass die Wirkstoffkette nicht überraschend aus der Einschublage in die Freilage herausspringen kann.

Die Selbstarretierung der Wirkstoffkette kann weiter gestärkt werden, wenn in einem Eingangsbereich der wenigstens einen Aussparung ein kranzartiges Haltemittel angeordnet ist, um in der Einschublage das Speichermittel zu fixieren. Bei dem kranzartigen Haltemittel kann es sich um eine Faser oder eine Hinterschneidung in der Aussparung handeln, welche ein Herausgleiten und/oder Herausrollen des Speichermittels der Wirkstoffkette aus der Aussparung verhindert.

Damit das Speichermittel formschlüssig in einer zylinderartigen Aussparung liegt, hat es sich als vorteilhaft, wenn ein Bodenbereich der wenigstens einen Aussparung halbkugelartig ausgestaltet ist. Das kugelartige Speichermittel kommt in dem halbkugelartig ausgestalteten Bodenbereich zum Liegen. Folglich bildet sich ein großflächiger Bereich, in dem ein Formschluss zwischen dem Speichermittel und der Aussparung entsteht. Über diesen Bereich des direkten formschlüssigen Kontaktes zwischen dem Speichermittel und der Aussparung wird dann jene Kraft übertragen, die von der Zugvorrichtung in die Wirkstoffkette eingebracht werden soll, wenn Letztere in der Einschublage angeordnet ist.

Eine weitere vorteilhafte Ausführungsvariante ist dadurch gekennzeichnet, dass die Speichermittel durch ein schnurartiges Mittel verbunden sind. Bei dem schnurartigen Mittel kann es sich um einen chirurgischen Stahldraht handeln, auf welchen die Speichermittel aufgepresst werden. Als vorteilhaft hat es sich dabei herausgestellt, wenn die Speichermittel aus einem PMMA-Kunststoff aufgebaut sind, welcher auf das schnurartige Mittel aufgepresst wird. Um eine entsprechende Wirkung zu generieren, wird der PMMA-Kunststoff des Speichermittels vorher mit einem medizinischen Wirkstoff, wie einem Antibiotikum, beladen.

die Ausführungsform des erfindungsgemäßen medizinischen Systems zeichnet sich dadurch aus, dass das Koppelelement wenigstens ein schlitzartiges Verbindungselement aufweiset, wobei das Verbindungselement in der Einschublage das schnurartige Mittel der Wirkstoffkette lagert. Das schlitzartige Verbindungselement kann in einer Ausführungsvariante als ein Ausschnitt in dem Koppelelement angeordnet sein. Es ermöglicht, dass in der Einschublage die Wirkstoffkette in die Aussparungen eingelegt werden kann. Das schnurartige Mittel ist jeweils in dem Verbindungselement angeordnet, so dass die Wirkstoffkette aus einem Ende des Koppelelementes herausragt. Durch diese Anordnung kann das Koppelelement in einer Linie mit der zu ziehenden Wirkstoffkette angeordnet sein, was ein einfaches Einbringen von Kraft in die Wirkstoffkette ermöglicht. Eine weitere Ausgestaltungsvariante zeichnet sich dadurch aus, dass das Verbindungselement zwei Aussparungen zumindest bereichsweise miteinander verbindet und/oder das Verbindungselement das Durchgangselement und wenigstens eine Aussparung zumindest bereichsweise miteinander verbindet. Bei dieser Variante stellt das Verbindungselement sicher, dass in jeder der Aussparungen ein Speichermittel gelagert werden kann, wobei das schnurartige Mittel auf kürzestem Wege zwischen den beiden Speichermitteln verlaufen kann. So ist ein gleichmäßiges Einbringen von Kraft auf die Wirkstoffkette möglich.

Eine weitere vorteilhafte Variante zeichnet sich dadurch aus, dass das Griffelement und das Koppelelement einstückig, bevorzugt einteilig, insbesondere materialeinheitlich sind, insbesondere das Griffelement und das Koppelelement T-artig zueinander angeordnet sind. Die Zugvorrichtung kann sowohl aus Metall, wie zum Beispiel aus chirurgischem Stahl, als auch aus üblichen Kunststoffen, wie zum Beispiel PMMA, Polyethylen, Polypropylen, Polyamid, Polyetherketon und Polysulfon, gefertigt sein. Die Formgebung kann durch Gießen, Spritzgießen oder auch durch konventionelle Metallbearbeitungsverfahren, wie zum Beispiel Schmieden und Fräsen, erfolgen.

Die oben genannte Aufgabe wird ebenfalls gelöst durch eine Zugvorrichtung zum Ziehen einer mit einem medizinischen Wirkstoff beladenen Wirkstoffkette, wobei die Zugvorrichtung ein Griffelement und ein Koppelelement aufweist, das Koppelelement, um die Wirkstoffkette zu ziehen, wenigstens eine Aussparung aufweist, in welche die zu ziehende Wirkstoffkette in einer Einschublag formschlüssig anordbar ist. Merkmale und Details, die dabei in Zusammenhang mit dem medizinischen System beschrieben wurden, gelten selbstverständlich auch für die erfindungsgemäße Zugvorrichtung.

Im Rahmen der Patentanmeldung wird ebenfalls eine Verwendung einer Zugvorrichtung zum Entfernen einer mit medizinischen Wirkstoffen beladenen Wirkstoffkette beansprucht. Dabei ist die Zugvorrichtung gemäß den oben gemachten Ausführungen ausgestaltet. Jedes Merkmal und Detail, welches in Zusammenhang mit der erfindungsgemäßen Zugvorrichtung bzw. dem medizinischen System aufgeführt wird, gilt dabei auch für die Verwendung der Zugvorrichtung.

Weitere Maßnahmen und Vorteile der Erfindung ergeben sich aus den Ansprüchen, der nachfolgenden Beschreibung und den Zeichnungen. In den Zeichnungen ist die Erfindung in mehreren Ausführungsbeispielen dargestellt. Es zeigen:
- Fig. 1: ein erfindungsgemäßes medizinisches System in einer Freilage,
- Fig. 2: das medizinische System in einer Einschublage,
- Fig. 3: eine schematische Schnittzeichnung durch die Zugvorrichtung der Fig. 1 entlang der Schnittlinie I-I,
- Fig. 4: eine schematische Schnittzeichnung durch die Zugvorrichtung entlang der Schnittlinie II-II und
- Fig. 5: eine weitere schematische Schnittzeichnung durch die Zugvorrichtung.

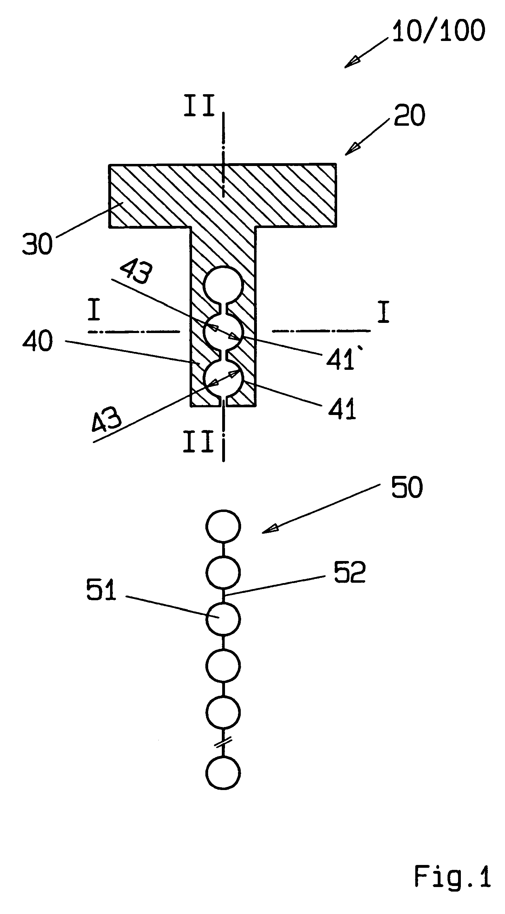

In Figur 1 ist ein medizinisches System 10 dargestellt, welches die oben genannte Aufgabe löst. Das medizinische System 10 weist eine Zugvorrichtung 20 und eine Wirkstoffkette 50 auf. Die Wirkstoffkette 50 besteht aus einer kettenartig aneinander gereihten Anordnung von Speichermitteln 51, welche in dem dargestellten Ausführungsbeispiel aus PMMA (Polymethylmethacrylat) bestehen. Diese Speichermittel 51 sind auf das schnurartige Mittel 52 aufgepresst worden. Bei dem schnurartigen Mittel 52 handelt es sich um einen Stahldraht. Die Speichermittel 51 sind beladen mit einem medizinischen Wirkstoff. Solcherart ausgestaltete Wirkstoffketten 50 werden als Wirkstoffträger genutzt, die zur Bekämpfung von Infektionen eines Knochengewebes genutzt werden. Dazu werden diese direkt in das zuvor chirurgisch sanierte und infizierte Gewebe eingelegt. Durch den direkten Kontakt der Wirkstoffkette 50 mit dem Bindegewebe und/oder dem Knochengewebe können die medizinischen Wirkstoffe direkt in diese eindringen. Allerdings müssen solcherart gestaltete Wirkstoffketten 50 nach einiger Zeit aus der Wunde wieder herausgezogen werden. Im Stand der Technik werden dazu vorzugsweise Zangen benutzt, die allerdings dazu führen, dass die Wirkstoffketten 50 zum Teil zerreißen.

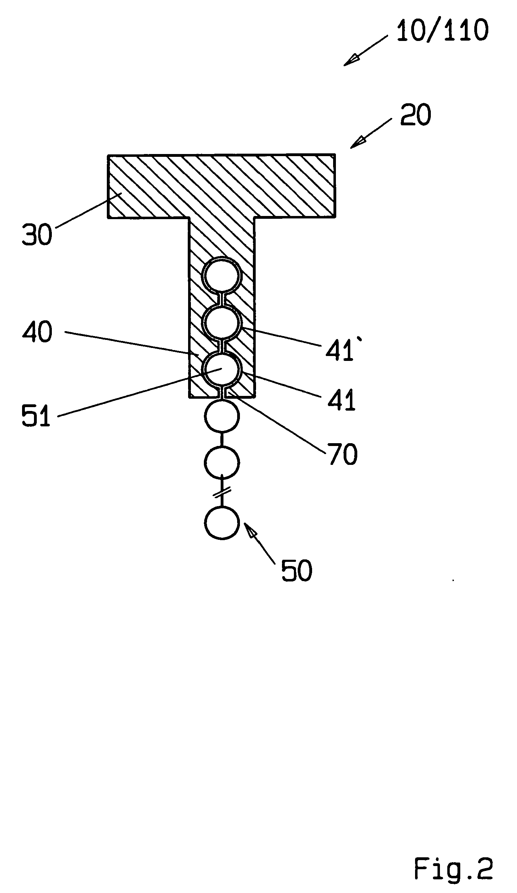

Um diesen Nachteil zu überwinden, wird im Rahmen der Erfindung eine Zugvorrichtung 20 beschrieben, die als Teil des medizinischen Systems 10 dazu dient, die Wirkstoffkette 50 aus der Wunde herauszuziehen. Um die Wirkstoffkette 50 aus einer Wunde zu ziehen, muss die Wirkstoffkette 50 aus der in Figur 1 gezeigten Freilage 100 in die in Figur 2 gezeigte Einschublage 110 überführt werden. In der Freilage 100 ist die Wirkstoffkette 50 getrennt von der Zugvorrichtung 20 angeordnet. Im Gegensatz dazu ist in der Einschublage 110 zumindest eines der Speichermittel 51 formschlüssig in einer Aussparung 41, 41' der Zugvorrichtung 20 angeordnet. Die Zugvorrichtung 20 weist ein Griffelement 30 und ein Koppelelement 40 auf. In dem dargestellten Ausführungsbeispiel sind das Griffelement 30 und das Koppelelement 40 T-artig zueinander angeordnet. In dem Koppelelement 40 sind zwei Aussparungen 41, 41' angeordnet. In diese Aussparungen 41,41' können die Speichermittel 51 eingelegt werden. Da die Speichermittel 51 im Allgemeinen eine kugelartige Form aufweisen, ist die Aussparung 41, 41' zylinderartig geformt, vorzugsweise durch eine Sackbohrung. Diese Ausgestaltung hat den Vorteil, dass beim Herausziehen der Wirkstoffkette aus der Wunde die Speichermittel 51 formschlüssig in den Aussparungen 41, 41' angeordnet sind. Somit ist die Fläche, über welche die Kraft aus der Zugvorrichtung 20 in die Wirkstoffkette 50 übertragen wird, groß. Somit sollte auch ein Durchmesser 43 der Aussparung nur leicht größer sein als der Durchmesser des Speichermittels 51. Dadurch ist sichergestellt, dass große Bereiche des Speichermittels 51 an den Wandungen der Aussparung 41, 41' anliegen.

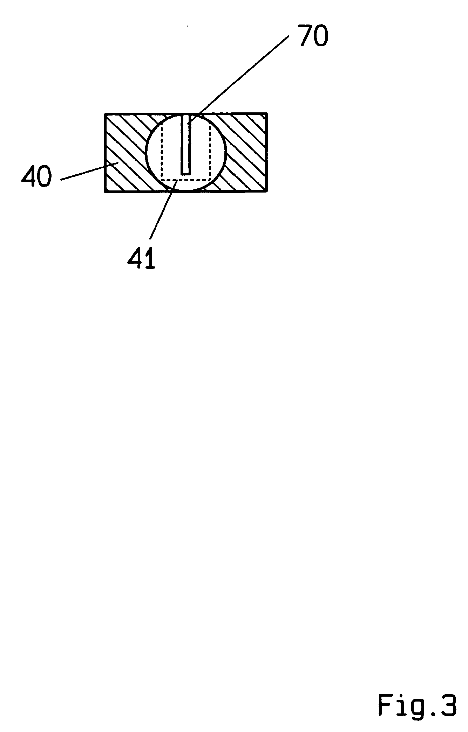

In Figur 3 ist eine prinzipielle Schnittzeichnung durch die Zugvorrichtung 20 entlang der Schnittlinie I - I aus Figur 1 dargestellt. Die Aussparung 41 ist zylinderartig ausgeformt und in das Koppelelement 40 eingebracht. Um das schnurartige Mittel 52 der Wirkstoffkette 50 zu lagern, ist jede der zwei Aussparungen 41, 41' mit einem Verbindungselement 70 versehen. Dieses Verbindungselement kann zwei Aussparungen 41, 41' zumindest bereichsweise miteinander verbinden oder eine Aussparung 41 mit einem Endbereich des Koppelelementes verbinden. Dabei lagert das Verbindungselement 70 in einer Einschublage das schnurartige Mittel 52. Das schnurartige Mittel 52 kann folglich aus dem Koppelelement 40 herausragen bzw. in der Einschublage 110 durch dieses hindurchgeführt werden.

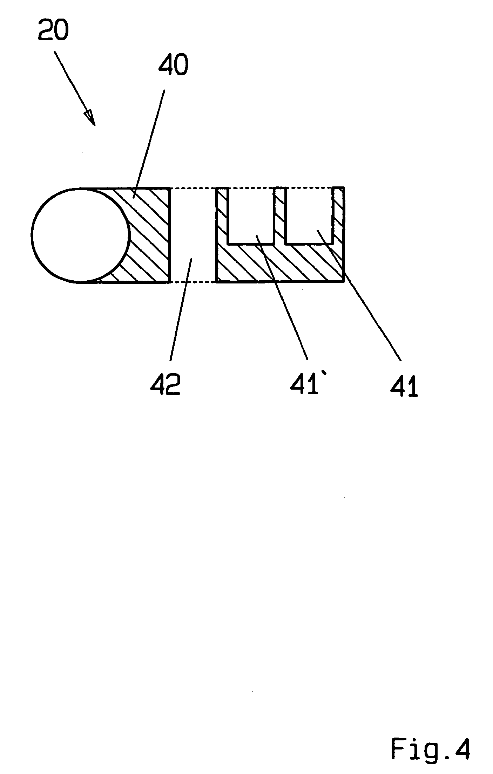

Die Figur 4 verdeutlicht ein Durchgangselement 42, welches ebenfalls in dem Koppelelement 40 angeordnet ist. Im Gegensatz zu den Aussparungen 41, 41' bildet das Durchgangselement 42 eine Durchführung in dem Koppelelement 40. Eine Wirkstoffkette 50 kann folglich durch das Durchgangselement 42 durchgefädelt werden. Erfindungsgemäß ist nun vorgesehen, dass mittels der Zugvorrichtung 20 die Wirkstoffkette 50 aus einer Wunde herausgezogen werden soll. Ist dieses zu einem Teil geschehen, kann ein Nutzer der erfindungsgemäßen Zugvorrichtung 20 jene abwinkeln, um so die Speichermittel 51 aus den Aussparungen 41, 41' herauszuführen. Im Anschluss ist es möglich, die Wirkstoffkette 50 durch das Durchgangselement 42 und die Zugvorrichtung 20 in Richtung der Wunde zu bewegen. Dann kann durch ein Abkippen der Zugvorrichtung 40 die Wirkstoffkette 50 aus der Freilage 100 wieder in die Einschublage 110 überführt werden. Der schon herausgezogene Rest der Wirkstoffkette 50 wird dabei durch das Durchgangselement 42 in Position gehalten und führt nicht zu einem versehentlichen Übergang der Wirkstoffkette aus der Einschublage in die Freilage. Dadurch wird die Sicherheit des erfindungsgemäßen medizinischen Systems 10 weiter erhöht.

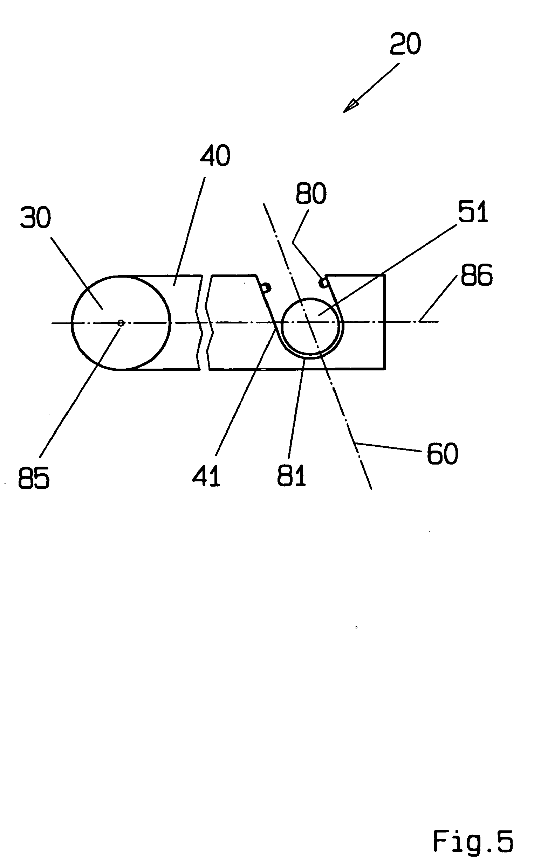

In Figur 5 ist die Ausgestaltung der erfindungsgemäßen Zugvorrichtung 10 dargestellt. In dem dargestellten Ausführungsbeispiel weist das Koppelelement 40 eine Aussparung 41 auf, deren Mittelachse 60 gegenüber einer von dem Griffelement 30 und dem Koppelelement 40 aufgespannten Ebene geneigt ist. Zur Verdeutlichung ist in dem dargestellten Ausführungsbeispiel eine Längsachse 85 des Griffelementes 30 und eine Längsachse 86 des Koppelelementes 40 dargestellt. Diese beiden Achsen spannen eine Ebene auf, gegenüber welcher die Mittelachse 60 der Aussparung 41 geneigt ist. Diese Neigung führt dazu, dass bei einem kraftvollen Herausziehen der Wirkstoffkette 50 das - hier schematisch angedeutete - Speichermittel 51 in Richtung eines Bodenbereiches 81 der Aussparung 41, 41' gezogen wird. Dieses führt zu einer Selbstarretierung der Wirkstoffkette 50 in der erfindungsgemäßen Zugvorrichtung 20. Weiterhin weist die Zugvorrichtung 20 ein kranzartiges Haltemittel 80 auf, das in einem Eingangsbereich der Aussparung 41 angeordnet ist. Das kranzartige Haltemittel 80 sorgt für eine Fixierung des Speichermittels 51 in der Aussparung 41. Um die kraftschlüssige Verbindung zwischen Speichermittel 51 und der Aussparung 41 zu verbessern, ist der Bodenbereich 81 in dem dargestellten Ausführungsbeispiel halbkugelartig ausgestaltet.

### Bezugszeichen

- 10: Medizinisches System

- 20: Zugvorrichtung

- 30: Griffelement

- 40: Koppelelement
- 41;41': Aussparung
- 42: Durchgangselement
- 43: Durchmesser Aussparung

- 50: Wirkstoffkette
- 51: Speichermittel
- 52: schnurartige Mittel

- 60: Mittelachse

- 70: Verbindungselement

- 80: Kranzartiges Haltemittel
- 81: Bodenbereich der Aussparung 41, 41'
- 85: Längsachse des Griffelementes 30
- 86: Längsachse des Koppelungselementes 40

- 100: Freilage
- 110: Einschublage

## Patentansprüche

1. Medizinisches System (10) mit einer Zugvorrichtung (20) und einer Wirkstoffkette (50), wobei
die Wirkstoffkette (50) mindestens zwei Speichermittel (51) aufweist, die kettenartig angeordnet sind,
wenigstens eines der Speichermittel (51) mit einem medizinischen Wirkstoff beladen ist,
die Zugvorrichtung (20) ein Griffelement (30) und ein Koppelelement (40) aufweist, und das Koppelelement (40) wenigstens eine Aussparung (41,41') aufweist,
in einer Freilage (100) die Wirkstoffkette (50) getrennt von der Zugvorrichtung (20) angeordnet ist,
in einer Einschublage (110) zumindest eines der Speichermittel (51) formschlüssig in der Aussparung (41,41') angeordnet ist, und
die Wirkstoffkette (50) reversibel aus der Frellage (100) in die Einschublage (110) überführbar ist,
**dadurch gekennzeichnet, dass**
das Griffelement (30) und das Koppelelement (40) einstückig und T-artig zueinander angeordnet , sind
wobei eine Mittenachse (60) der wenigstens einen Aussparung (41,41') gegenüber einer von dem Griffelement (30) und dem Koppelelement (40) aufgespannten Ebene geneigt ist, und
dass die Speichermittel (51) durch ein schnurartiges Mittel (52) verbunden sind, und
das Koppelelement (40) wenigstens ein schlittzartiges Verbindungselement aufweist. wobei das Verbindungselement in der Einschublage das schnurartige Mittel (52) lagert.

2. Medizinisches System (10) nach Anspruch 1, **dadurch gekennzeichnet, dass** die wenigstens eine Aussparung (41,41') zylinderartig ausgeformt ist.

3. Medizinisches System (10) nach einem der Ansprüche 1 oder 2, **dadurch gekennzeichnet, dass** ein Durchmesser der wenigstens einen Aussparung (41,41') größer ist als der Durchmesser eines Speichermittels (51).

4. Medizinisches System (10) nach einem der vorhergehenden Ansprüche, **dadurch gekennzeichnet, dass** das Koppelelement (40) ein Durchgangselement (42) aufweist, welches eine Durchführung in dem Koppelelement (40) bildet.

5. Medizinisches System (10) nach Anspruch 4, **dadurch gekennzeichnet, dass** das Durchgangselement (42) einen Durchmesser aufweist, der größer ist als der Durchmesser der wenigstens einen Aussparung (41,41').

6. Medizinisches System (10) nach einem der vorhergehenden Ansprüche, **dadurch gekennzeichnet, dass** die Mittelachse (60) gegenüber der aufgespannten Ebene In Richtung des Griffelement (30) geneigt ist.

7. Medizinisches System (10) nach einem der vorhergehenden Ansprüche, **dadurch gekennzeichnet, dass** in einem Eingangsbereich der wenigstens einen Aussparung (41,41') ein kranzartiges Haltemittel angeordnet ist, um in der Einschublage (110) das Speichermittel (51) zu fixieren.

8. Medizinisches System (10) nach einem der vorhergehenden Ansprüche, **dadurch gekennzeichnet, dass** ein Bodenbereich der wenigstens einen Aussparung (41,41') halbkugelartig ausgestaltet ist.

9. Medizinisches System (10) nach einem der Ansprüche 4 bis 8, **dadurch gekennzeichnet, dass** das Durchgangselement (42) zwischen der wenigstens einen Aussparung (41,41') und dem Griffelement (30) angeordnet ist.

10. Medizinisches System (10) nach einem der vorhergehenden Ansprüche, **dadurch gekennzeichnet, dass** das Verbindungselement zwei Aussparungen (41,41') zumindest bereichsweise miteinander verbindet und/oder das Verbindungselement das Durchgangselement (42) und wenigstens eine Aussparung (41,41') zumindest bereichsweise miteinander verbindet.

11. Medizinisches System (10) nach einem der vorhergehenden Ansprüche, **dadurch gekennzeichnet, dass** das Griffelement (30) und das Koppelelement (40) einstückig, bevorzugt einteilig, insbesondere materialeinheitlich sind.

## Claims

1. Medical system (10) having a pulling device (20) and an active substance chain (50), whereby
the active substance chain (50) comprises at least two storage means (51) that are arranged in a chain-like manner;
at least one of the storage means (51) is loaded with an active medical substance; the pulling device (20) comprises a grip element (30) and a coupling element (40), and the coupling element (40) comprises at least one recess (41, 41');
in a free position (100), the active substance chain (51) is arranged such as to be separate from the pulling device (20):
in an insertion position (110), at least one of the storage means (51) is arranged in the recess (41, 41') in a form-fitting manner, and
the active substance chain (50) can be transferred reversibly from the free position (100) to the insertion position (110);
**characterised in that**
the grip element (30) and the coupling element (40) are provided as the same part and are arranged in a T-like manner with respect to each other;
whereby a central axis (60) of the at least one recess (41, 41') is inclined with respect to a plane formed by the grip element (30) and the coupling element (40);
and
**in that** the storage means (51) are connected through a cord-like means (52); and
the coupling element (40) comprises at least one slit-like connecting element,
whereby the connecting element stores the cord-like means (52) in the insertion position.

2. Medical system (10) according to claim 1, **characterised in that** the at least one recess (41, 41') is shaped to be cylinder-like.

3. Medical system (10) according to any of the claims 1 or 2, **characterised in that** a diameter of the at least one recess (41, 41') is larger than the diameter of a storage means (51).

4. Medical system (10) according to any one of the preceding claims, **characterised in that** the coupling element (40) comprises a through element (42) that forms a feed-through in the coupling element (40).

5. Medical system (10) according to claim 4, **characterised in that** the through element (42) has a diameter that is larger than the diameter of the at least one recess (41, 41').

6. Medical system (10) according to any one of the preceding claims, **characterised in that** the central axis (60) is inclined in the direction of the grip element (30) with respect to the plane that is formed.

7. Medical system (10) according to any one of the preceding claims, **characterised in that** a wreath-like holding element is arranged in the entry region of the at least one recess (41, 41') in order to fix the storage means (51) in place in the insertion position (110).

8. Medical system (10) according to any one of the preceding claims, **characterised in that** a floor region of the at least one recess (41, 41') is designed to be semispherical in shape.

9. Medical system (10) according to any one of the claims 4 to 8, **characterised in that** the through element (42) is arranged between the at least one recess (41, 41') and the grip element (30).

10. Medical system (10) according to any one of the preceding claims, **characterised in that** the connecting element connects two recesses (41, 41') to each other, at least regions thereof, and/or the connecting element connects the through element (42) and at least one recess (41, 41') to each other, at least regions thereof.

11. Medical system (10) according to any one of the preceding claims, **characterised in that** the grip element (30) and the coupling element (40) are the same piece, preferably as just one part, in particular are made of the same material.

## Revendications

1. Système médical (10) avec un dispositif de traction (20) et une chaîne de substances actives (50), dans lequel
la chaîne de substances actives (50) présente au moins deux moyens de stockage (51) qui sont disposés à la manière d'une chaîne.
au moins un des moyens de stockage (51) est chargé avec une substance active médicale,
le dispositif de traction (20) présente un élément de saisie (30) et un élément de raccord (40), et l'élément de raccord (40) présente au moins un évidement (41, 41'),
la chaîne de substances actives (50) est disposée séparément du dispositif de traction (20) dans une position libre (100),
au moins un des moyens de stockage (51) est disposé par conjugaison de forme dans l'évidement (41, 41') dans une position insérable (110), et
la chaîne de substances actives (50) peut être transférée de manière réversible de la position libre (100) dans la position insérable (110),
**caractérisé en ce que**
l'élément de saisie (30) et l'élément de raccord (40) sont disposés d'une pièce et à la manière d'un T l'un par rapport à l'autre,
dans lequel un axe médian (60) de l'au moins un évidement (41, 41') est incliné par rapport à un plan fixé par élément de saisie (30) et l'élément de raccord (40), et que les moyens de stockage (51) sont reliés par un moyen de type cordon (52), et l'élément de raccord (40) présente au moins un élément de connexion de type fente, dans lequel l'élément de connexion loge le moyen de type cordon (52) dans la position insérable.

2. Système médical (10) selon la revendication 1, **caractérisé en ce que** l'au moins un évidement (41, 41') est réalisé de manière cylindrique.

3. Système médical (10) selon l'une quelconque des revendications 1 ou 2, **caractérisé en ce qu'**un diamètre de l'au moins un évidement (41, 41') est supérieur au diamètre d'un moyen de stockage (51).

4. Système médical (10) selon l'une quelconque des revendications précédentes, **caractérisé en ce que** l'élément de raccord (40) présente un élément de passage (42) qui forme une traversée dans l'élément de raccord (40).

5. Système médical (10) selon la revendication 4, **caractérisé en ce que** l'élément de passage (42) présente un diamètre qui est supérieur au diamètre de l'au moins un évidement (41, 41').

6. Système médical (10) selon l'une quelconque des revendications précédentes, **caractérisé en ce que** l'axe médian (60) est incliné en direction de l'élément de saisie (30) par rapport au plan fixé.

7. Système médical (10) selon l'une quelconque des revendications précédentes, **caractérisé en ce qu'**un moyen de maintien de type couronne est disposé dans une zone d'entrée de l'au moins un évidement (41, 41') pour fixer le moyen de stockage (51) dans la position insérable (110).

8. Système médical (10) selon l'une quelconque des revendications précédentes, **caractérisé en ce qu'**une zone de fond de l'au moins un évidement (41, 41') est configurée de manière hémisphérique.

9. Système médical (10) selon l'une quelconque des revendications 4 à 8, **caractérisé en ce que** l'élément de passage (42) est disposé entre l'au moins un évidement (41, 41') et l'élément de saisie (30).

10. Système médical (10) selon l'une quelconque des revendications précédentes, **caractérisé en ce que** l'élément de connexion relie deux évidements (41, 41') ensemble au moins par région et/ou l'élément de connexion relie l'élément de passage (42) et au moins un évidement (41, 41') ensemble au moins par région.

11. Système médical (10) selon l'une quelconque des revendications précédentes, **caractérisé en ce que** l'élément de saisie (30) et l'élément de raccord (40) sont d'une pièce, de préférence en une partie, notamment unitaires au niveau du matériau.
